(19) Europäisches Patentamt
European Patent Office
Office européen des brevets

(11) Veröffentlichungsnummer: **0 000 220**
**B1**

# (12) EUROPÄISCHE PATENTSCHRIFT

(45) Veröffentlichungstag der Patentschrift: **29.04.81**

(21) Anmeldenummer: **78200041.8**

(22) Anmeldetag: **14.06.78**

(51) Int. Cl.³: **C 07 D 403/00, A 61 K 31/495**

(54) **Dihydrouracile, Verfahren zu ihrer Herstellung und sie enthaltende Arzneimittel.**

(30) Priorität: **18.06.77 DE 2727469**

(43) Veröffentlichungstag der Anmeldung:
**10.01.79 Patentblatt 79/1**

(45) Bekanntmachung des Hinweises auf die Patenterteilung:
**29.04.81 Patentblatt 81/17**

(84) Benannte Vertragsstaaten:
**BE CH DE FR GB LU NL SE**

(56) Entgegenhaltungen:
**DE - A - 2 242 382**

(73) Patentinhaber: **HOECHST AKTIENGESELLSCHAFT**
**Zentrale Patentabteilung Postfach 80 03 20**
**D-6230 Frankfurt/Main 80 (DE)**

(72) Erfinder: **Weber, Rolf-Ortwin, Dr.**
**Erbsenacker 18**
**D-6200 Wiesbaden-Naurod (DE)**
Erfinder: **Anagnostopulos, Hiristo**
**Kantstrasse 22**
**D-6204 Taunusstein 01 (DE)**
Erfinder: **Gebert, Ulrich, Dr.**
**Albert-Lortzing-Strasse 2**
**D-6233 Kelkheim/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 220 B1

Dihydrouracile, Verfahren zu ihrer Herstellung und sie enthaltende Arzneimittel

Die vorliegende Erfindung betrifft Dihydrouracile ein schließlich ihrer Salze, Verfahren zu deren Herstellung und Arzneimittel, die diese Verbindungen als Wirkstoffe enthalten.

Aus der deutschen Offenlegungsschrift 2.242.382 sind bereits Piperazinverbindungen bekannt, die neben einer Trifluormethylphenylgruppe auch noch einen Dihydrouracilrest tragen können. Über ihre pharmakologische Wirkung wird jedoch nichts berichtet. Die dort beschriebenen analogen Succinimidverbindungen haben lediglich appetithemmende Eigenschaften. Es war deshalb nicht vorauszusehen, daß Derivate derartiger Dihydrouracile, die am Phenylring keine Trifluormethylgruppe tragen und am Dihydrouracilring Alkylsubstituenten aufweisen, völlig andere pharmakologische Wirkungen zeigen.

Es ist bekannt, daß Pizotifen ebenso wie Methysergid aufgrund der serotoninantagonistischen Wirkung als Antimigränemittel verwendet wird (H. Heyck "Med. Welt" Band 25, (1974) Seiten 1853 bis 1874). Kürzlich wurde auch über Erfolge bei der Migränebehandlung mit dem Gefäßtherapeutikum Bencyclan berichtet (M. Abel, "Med. Welt" Band 27, (1976) Seiten 1509 bis 1512), das neben einem schwachen Serotoninantagonismus insbesondere die Eigenschaft besitzt, die Aggregation von Thrombozyten zu hemmen.

Es wurde nun gefunden, daß Dihydrouracile der Formel I

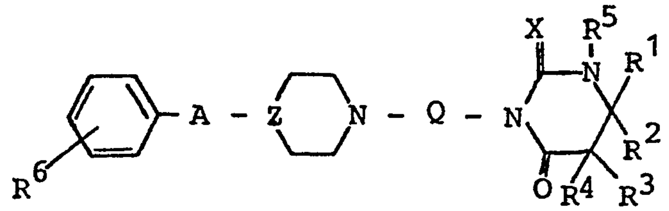

(I)

worin

$R^1$ Alkyl mit 1 bis 2 C-Atomen,

$R^2$ bis $R^5$ Wasserstoff oder Alkyl mit bis zu 2 C-Atomen, wobei $R^2$ bis $R^5$ gleich oder verschieden sind,

$R^6$ Wasserstoff, einen anellierten Benzolring oder 1 bis 3 Substituenten der Gruppe Alkoxy mit 1 bis 3 C-Atomen, Halogen oder Alkyl mit 1 bis 4 C-Atomen,

A eine Einfachbindung oder die Gruppe

$$C_6H_5-\overset{|}{\underset{|}{C}}H,$$

Q unverzweigtes oder verzweigtes Alkylen mit 2 bis 6 C-Atomen, wobei mindestens 2 C-Atome zwischen den beiden Stickstoffatomen stehen, oder die 2-Hydroxy-1,3-propylen-Gruppe

X Sauerstoff oder Schwefel und

Z Stickstoff oder eine Gruppe

$$-\overset{|}{\underset{|}{C}}H$$

neben anderen wertvollen pharmakologischen Eigenschaften bei guter Verträglichkeit vor allem hochwirksame Serotoninantagonisten und gleichzeitig starke Thrombozytenaggregationshemmer darstellen. Sie kommen deshalb besonders für die Migränetherapie in Betracht.

Gegenstand der Erfindung sind somit Verbindungen der Formel (I) einschließlich ihrer Additionssalze mit physiologisch verträglichen Säuren.

Außer den bereits erwähnten wertvollen pharmakologischen Eigenschaften zeigen die erfindungsgemäßen Verbindungen antihistaminische Aktivität, eine Erhöhung der Erythrozytenfluidität, zum Teil auch psychotrope Wirkung, einen schwachen Bradykininantagonismus sowie blutdrucksenkende Effekte.

Besonders interessant sind Verbindungen der Formel (I), in denen

$R^1$ Alkyl mit 1 bis 2 C-Atomen,

$R^2$,$R^3$ Wasserstoff oder Alkyl mit 1 bis 2 C-Atomen,

$R^4$,$R^5$ Wasserstoff,

$R^6$ Wasserstoff oder Halogen,

A eine Einfachbindung,

Q eine unverzweigte Alkylenkette mit 2 bis 3 C-Atomen,
X Sauerstoff oder Schwefel und
Z Stickstoff bedeuten.

Hiervon sind solche besonders bevorzugt, in denen $R^1$ Methyl, einer der Reste $R^2$ und $R^3$ Methyl und der andere Wasserstoff und $R^6$ Wasserstoff oder Fluor in 4-Stellung bedeutet, wie die Verbindungen Nr. 8, 10 und 14 bis 16 der Tabelle IV.

Geeignete Gruppierungen

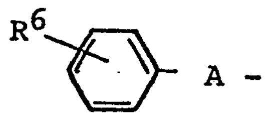

sind beispielsweise Phenyl, 2-, 3- oder 4-Tolyl, 2,6- oder 3,4-Dimethylphenyl, 2-, 3- oder 4-Methoxyphenyl, Naphthyl, Fluorphenyl, Chlorphenyl, Bromphenyl oder Diphenylmethyl.

Geeignete Gruppierungen

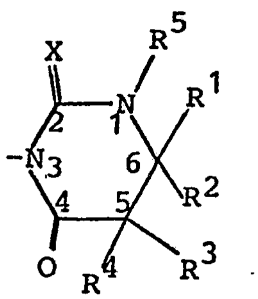

sind beispielsweise 5,6- oder 6,6-Dimethyl-dihydrouracil-3-yl; 5,6- oder 6,6-Dimethyl-2-thio-dihydrouracil-3-yl; 1,6,6-Trimethyl-2-thio-dihydrouracil-3-yl; 1,6,6-Trimethyl-dihydrouracil-3-yl; 6-Methyl-dihydrouracil-3-yl.

Geeignete Brückenglieder Q stellen beispielsweise Äthylen, Propylen, Butylen, Pentylen, Hexylen und 2-Hydroxypropylen dar, wobei die Reste mit mindestens 3 C-Atomen auch verweigt sein können, so daß noch mindestens 2 C-Atome in der Kette stehen.

Gegenstand der Erfindung ist auch ein Verfahren zur Herstellung von Dihydrouracilen der Formel (I), bei dem man eine Verbindung der Formel II

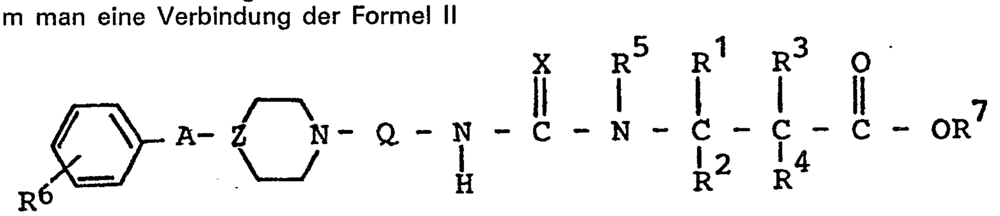 (II)

in der $R^1$ bis $R^6$, A, Q, Z und X die oben genannten Bedeutungen haben und $OR^7$ Hydroxyl oder eine Alkoxygruppe mit 1 bis 4 C-Atomen bedeutet, unter Abspaltung von $R^7OH$ cyclisiert und gewünschtenfalls die Verbindungen der Formel I als solche isoliert oder mit Säuren in ihre physiologisch verträglichen Säureadditionssalze umwandelt.

Die Cyclisierung der Verbindung II kann auf verschiedene Weise nach üblichen Kondensationsmethoden erfolgen, vor allem in einem geeigneten Lösungsmittel und/oder in Gegenwart von Mineralsäuren und/oder durch Erhitzen des bei der Synthese der Verbindung II anfallenden Reaktionsgemisches bzw. der daraus isolierten Reinsubstanz II. Verbindungen, in denen $R^7$ für Wasserstoff steht, lassen sich vielfach besonders gut mit Hilfe von Mineralsäuren, wie Schwefelsäure oder Halogenwasserstoffsäuren, vorzugsweise von Salzsäure oder Bromwasserstoffsäure in einem Alkohol wie Methanol, Äthanol, Propanol oder Isopropanol cyclisieren. Die Cyclisierung kann aber auch, was bevorzugt ist, durch Erhitzen der reinen Substanzen unter Abspaltung von Wasser oder ohne Lösungsmittel- und Säurezusatz mittels geeigneter Dehydratisierungsmittel, wie Acetylchlorid oder Essigsäureanhydrid, erzielt werden. Zweckmäßig wird am Siedepunkt des entsprechenden Lösungs- oder Kondensationsmittels gearbeitet.

Die Cyclisierung von Verbindungen, in denen $R^7$ Alkyl ist, wird bevorzugt in einem geeigneten Lösungsmittel und/oder in Gegenwart von Mineralsäuren, z.B. der obengenannten Säuren und Alkohole, durchgeführt. Bei Verbindungen, in denen X Schwefel ist, ist die Cyclisierung in Lösungsmitteln ohne Zusatz von Mineralsäuren besonders bevorzugt. Es ist auch möglich, Verbindungen der Formel II,

in denen $R^7$ Alkyl mit 1 bis 4 C-Atomen bedeutet, zunächst mit einem Alkalihydroxyd zu einer Verbindung der Formel II, in der $R^7$ Wasserstoff bedeutet, zu hydrolysieren und diese anschließend zu cyclisieren. Diese Hydrolyse kann mit Vorteil in wässerigem oder wässerig-acetonischem oder wässerig alkoholischem Medium durchgeführt werden, wobei der Alkohol zweckmäßig 1 bis 3 C-Atome hat.

Die Darstellung der Ausgangssubstanzen der Formel II ist auf verschiedene Weise möglich. So gewinnt man Verbindungen, in denen $R^5$ Wasserstoff ist,

a) durch Umsetzung eines Amins der Formel III

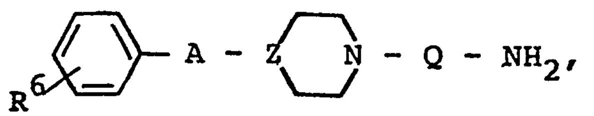 III

mit einem 3-Isocyanato- bzw. 3-Isothiocyanato-alkancarbonsäureester der Formel IV

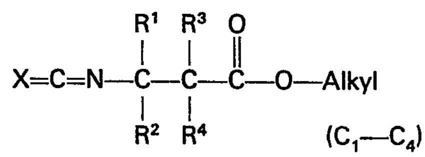 IV

oder Verbindungen der Formel II, in denen $R^5$ Wasserstoff oder Alkyl bedeutet, indem man

b) ein Amin der Formel III zuerst mit einer Verbindung der Formel V

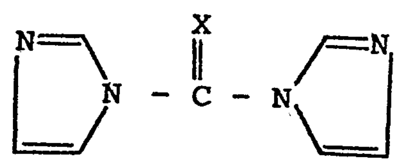 V

zu einer Verbindung der Formel VI

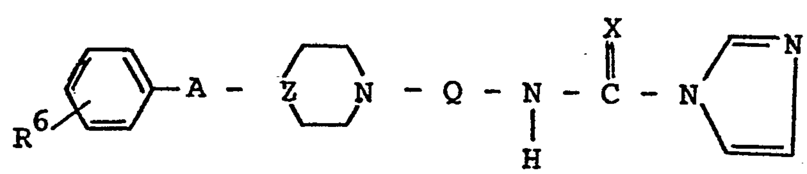 VI

und diese anschließend nach oder vorzugsweise ohne Zwischenisolierung mit einem 3-Amino-alkancarbonsäureester der Formel VII

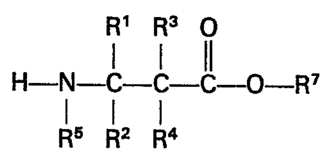 VII

als solchem oder in Form eines Säureadditionssalzes umsetzt, oder

c) ein Amin der Formel III mit einem Carbamoylhalogenid der Formel VIII

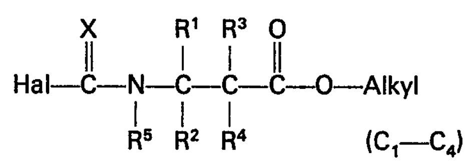 VIII

worin Hal vorzugsweise Chlor ist, zu einem Hydrohalogenid einer Verbindung der Formel II umsetzt.

Geeignete Ausgangsstoffe der Formel (III) sind beispielsweise 1-Phenyl-4-(3-aminopropyl)-piperazin, 1-(2-Methylphenyl)- und 1-(3-Methylphenyl)-4-(3-aminopropyl)-piperazin, 1-(2-Methoxyphenyl)-, 1-(3-Methoxyphenyl)- und 1-(4-Methoxyphenyl)-4-(3-aminopropyl)-piperazin, 1-(3-Dimethylphenyl)- und 1-(2,6-Dimethylphenyl)-4-(3-aminopropyl)-piperazin, 1-(1-Naphthyl)- und 1-(4-Fluorphenyl)-4-(3-aminopropyl)-piperazin, 1-Phenyl-4-(2-aminoäthyl)-piperazin, 1-(2-Methoxyphenyl)-4-(2-aminoäthyl)-piperazin, 1-(4-Methoxyphenyl)-4-(5-aminopentyl)-piperazin, 1-Diphenylmethyl-4-(3-aminopropyl)-piperazin, 4-Phenyl-1-(3-aminopropyl)-piperidin, 1-Phenyl-4-(4-aminobutyl)-piperazin, 1-Phenyl-4-(5-aminopentyl)-piperazin, oder 4-Phenyl-1-(2-aminoäthyl)-piperidin.

Geeignete 3-Isocyanato- bzw. Isothiocyanatoalkancarbonsäureester der Formel (IV) sind beispielsweise 3-Isocyanatoisovaleriansäuremethylester und -äthylester, 3-Isocyanatoisovaleriansäure-n- und isopropylester, die verschiedenen 3-Isocyanatoisovaleriansäurebutylester, 3-Isothiocyanatoisovaleriansäuremethylester und -äthylester, 3-isothiocyanatoisovaleriansäure-n- und -isopropylester, die verschiedenen 3-Isothiocyanatoisovaleriansäurebutylester, 2-Methyl-3-isocyanatobuttersäuremethylester und 2-Methyl-3-isothiocyanatobuttersäuremethylester.

Geeignete 3-Aminoalkancarbonsäure-Derivate der Formel (VII) sind zum Beispiel solche, die den oben genannten Isocyanatoverbindungen entsprechen und in 3-Stellung statt der Isocyanatogruppe eine Aminogruppe enthalten, wie 3-Aminoisovaleriansäuremethylester, 3-Amino-2-methyl-buttersäuremethylester und 3-Methylaminoisovaleriansäuremethylester.

Bei Verfahrensweise a) arbeitet man zweckmäßig in gegenüber den Reaktionspartnern inerten Lösungsmitteln wie Xylol, Toluol, Mesitylen, Benzol, Methylenchlorid oder Chloroform, und zwar bevorzugt beim Siedepunkt des Lösungsmittels; die Reaktion kann jedoch auch bei Raum-temperatur durchgeführt werden, wobei sich das Reaktionsgemisch in der Regel von selbst erwärmt. Dann wird das Lösungsmittel, zweckmäßig unter vermindertem Druck, entfernt und die Zwischenverbindung II entweder nach Reinigung durch Umkristallisation oder direkt als Rohprodukt — wie vorstehend beschrieben — in wässerigem oder wässerig-alkoholischem Medium, vorzugsweise an dessern Siedepunkt, im Falle von X = Sauerstoff vorzugsweise in Gegenwart von Mineralsäuren cyclisiert. Im Falle von X = Schwefel ist in der Regel ein Säurezusatz nicht notwendig. Auch eine Cyclisierung durch Erhitzen auf Temperaturen von über 150°C, vorzugsweise um 200°C und unter Schutzgas ist möglich.

Die Verfahrensvarianten b) und c) lassen sich im allgemeinen bei Raumtemperatur und zweckmäßig als Eintopfverfahren durchführen. Bei Verfahren (b), das besonders gut in Tetrahydrofuran abläuft, wird nach Zusatz der Verbindung VII die weitere Umsetzung und die zuvor beschriebene Cyclisierung jedoch vorteilhaft am Siedepunkt des Reaktionsgemisches durchgeführt. Verbindungen der Formel II, in denen $R^7$ Wasserstoff ist, werden bevorzugt — nachdem sie in reiner Form isoliert sind — durch Erhitzen ohne Lösungsmittel cyclisiert, und zwar vorteilhaft bei Temperaturen über 150°C, vorzugsweise bei etwa 200°C und unter Schutzgas. Beim Einsatz von im Reaktionsgemisch nur schwer löslichen Verbindungen der Formel VII ist zur Erzielung der gewünschten Umsetzung der Zusatz eines gegenüber den Reaktionsteilnehmern inerten, polaren Lösungsmittels wie Dimethylformamid zweckmäßig, damit eine homogene Lösung erhalten wird.

Zur Herstellung der Mono- oder Bisadditionssalze, die nach den allgemein üblichen Methoden erfolgt, sind physiologisch verträgliche Mineral- oder Sulfonsäuren geeignet, wie Chlorwasserstoffsäure, Bromwasserstoffsäure, Schwefelsäure, Benzolsulfonsäure, Methansulfonsäure, p-Toluolsulfonsäure und Cyclohexylsulfaminsäure.

Die Stabilität der erfindungsgemäß erhaltenen Verbindungen, die im allgemeinen kristallin sind, erlaubt die Herstellung von Arzneimittelzubereitungen für orale, parenterale und rektale Verabreichung. Die Herstellung dieser Zubereitungen kann nach der üblichen Praxis durch Zumischen passender und verträglicher Hilfsstoffe, wie Stärke, Milchzucker, Cellulosederivate, Stearinsäure oder ihre Salze, Lösungsmittel, Lösungsvermittler, Zäpfchenmasse, Trägerstoffe wie Chloride, Phosphate und Carbonate in üblicher Weise zu Pulvern, Tabletten, Dragees, Kapseln, Zäpfchen, Lösungen, Pasten oder Suspensionen erfolgen. Auch eine Verarbreichung der reinen Substanzen in Form von Mikrokapseln ist möglich und ebenso auch eine Kombination mit anderen Wirkstoffen.

Ihre Dosierung beim Menschen ist 0,1 bis 50 mg, vorzugsweise 0,5 bis 10 mg, insbesondere 0,5 bis 3 mg per os pro Tag.

Pharmakologische Prüfung und Ergebnisse

*1. Serotoninantagonistische Wirkung*

Zur Charakterisierung der serotoninantagonistischen Wirkung wurden folgende pharmakologische Testmodelle verwendet:

*Test a:* Versuchsanordnung nach Konzett und Rössler (Arch. exp. Path. Pharmak. *195* (1940) 71): Albino-Meerschweinchen aus hauseigener Zucht werden mit 1,25 g/kg Urethan intraperitoneal (i.p.) narkotisiert. Die Registrierung des Bronchialwiderstandes erfolgt bei geöffnetem Thorax über ein Aufnahmegerät ("Broncho-transducer") der Firma Ugo Basile (Mailand); die Atem-frequenz wird durch künstliche Beatmung mit einer Frequenz von 58 Huben/min konstant gehalten, der Aufblasdruck beträgt 9 cm $H_2O$-Säule. Zur Erzeugung der Bronchospasmen injiziert man alle 15 min 5 bis 10 $\mu$g/kg Serotonin (als Serotonin-kreatinin-sulfat) in einem Volumen von 0,1 ml/kg Körpergewicht (KG) in eine der Jugularvenen. Die Prüfsubstanzen werden in einem Volumen von 1 ml/kg entweder intravenös innerhalb von 30 s 2 min vor der Erzeugung des Serotoninspasmus oder aber intraduodenal bzw. oral mittels Schlundsonde 15 min vor dem Spasmus verabreicht Der $ED_{50}$-Wert entspricht der Dosis, durch die ein Serotonin-Spasmus 2 min nach i.v.-Injektion bzw. 15 min nach oraler oder intraduodenaler

Gabe des Präparates um 50% gehemmt wird (seihe Tabelle I).

*Test b:* Serotoninantagonismus am isolierten Uterus der Ratte nach Robert A. Turner: Screening Methods in Pharmacology, Academic Press 1965 (New York and London): Weibliche Sprague-Dawley-Ratten mit einem Gewicht von 180 bis 240 g erhalten zur Erzeugung des Oestrus 24 Stunden vor der Uterusentnahme 2 mg/kg Oestromon (Firma Merck, Darmstadt) intraperitoneal. Das auf 22°C gehaltene Organbad enthält eine Tyrode-Lösung mit spezifischer Zusammensetzung. Das Präparat wird in wäßriger Lösung mit einem Gesamtvolumen von 0,1 ml in das Bad appliziert. Die verabreichte Serotonin-Konzentration beträgt $10^{-8}$ g/ml Bad. Der $ED_{50}$-Wert entspricht der Dosis, durch die ein Serotonin-Spasmus um 50% gehemmt wird (siehe Tabelle 1).

*Test c:* 5-Hydroxyl-L-tryptophan-Antagonismus an der Maus: Eine einmalige Gabe von 5-Hydroxy-L-tryptophan bewirkt bei Mäusen eine starke Erhöhung der Defakation aufgrund der gesteigerten Biosynthese von Serotonin aus der exogen zugeführten Aminosäure. Durch Serotoninantagonisten läßt sich dieser Defäkationseffekt vermindern. Die Prüfsubstanzen werden 45 min vor der Tryptophan-Gabe oral verabreicht. Die Tryptophan-Dosis beträgt 40 mg/kg i.p.

Die Beurteilung erfolgt 1 Stunde nach Verabreichung des Präparates durch Messung de ausgeschiedenen Kotmenge. Eine Reduzierung dieser Menge um<25% der Leerkontrolle wird je nach dem Ergebnis mit 0 oder (+), von 25 bis 50% mit +, von 50 bis 75% mit ++ und >75% mit +++ angegeben (siehe Tabelle I).

TABELLE I

| Test | Substanz-Nr. | Art der Verabreichung | $ED_{50}$ in $\mu g/kg$ | n |
|---|---|---|---|---|
| a | 8 | i.v. | 3 – 10 | 5 |
| | | i.d. | 10 – 30 | 3 |
| | | p.o. | 100 – 300 | 3 |
| | 10 | i.v. | 10 – 30 | 2 |
| | 14 | i.v. | 10 – 30 | 3 |
| | | i.d. | 300 | 2 |
| | | p.o. | ca. 300 | 2 |
| | 15 | i.v. | 1 – 3 | 6 |
| | | i.d. | 10 – 30 | 2 |
| | 16 | i.v. | 1 – 3 | 7 |
| | | i.d. | ca. 30 | |
| | Pizotifen-HCl | i.v. | 10 – 50 | 2 |
| | | i.d. | 10 – 100 | 2 |
| | | p.o. | 500 – 1000 | 2 |
| **Test** | **Substanz-Nr.** | | **$ED_{50}$ in $\mu g/ml$ Bad** | **n** |
| b | 8 | | 0,001 – 0,01 | 3 |
| | 14 | | 0,001 – 0,01 | 3 |
| | 15 | | 0,001 – 0,01 | 4 |
| | 16 | | 0,0001–0,001 | 4 |
| | Pizotifen-HCl | | 0,001 – 0,01 | 4 |

Abkürzungen:

i.v. = intravenös

i.d. = intraduodenal

p.o. = per os

n = Zahl der Tiere

TABELLE I (Fortsetzung)

| Test | Substanz-Nr. | Dosis in mg/kg p.o. | Wirkungsstärke | n |
|---|---|---|---|---|
| | 14 | 10 | + | 10 |
| | | 20 | + | 10 |
| c | 15 | 10 | + | 10 |
| | | 20 | ++ | 10 |
| | 16 | 10 | ++ | 10 |
| | | 20 | ++ | 10 |
| | Pizotifen-HCl | 20 | + | 30 |

Die serotominantagonistische Wirking der nachfolgenden Verbindungen wurde nach dem Modell von Konzett-Rössler (Test a) und die $LD_{50}$ Werte an der Maus bestimmt:

TABELLE I (a)

| Substanz-Nr. | $ED_{50}$ in mg/kg i.v. | $LD_{50}$ (Maus) in mg/kg i.p. |
|---|---|---|
| 1 | 0,03 – 0,1 | 100 – 200 |
| 4 | 0,1 – 0,3 | 300 – 1000 |
| 5 | ~ 0,3 | 100 – 300 |
| 6 | 0,01 – 0,03 | 100 – 300 |
| 9 | 0,03 – 0,3 | 100 – 300 |
| 17 | 0,01 – 0,1 | 300 – 600 |
| 21 | 0,03 – 0,1 | >150 |
| 22 | ~ 0,01 | 150 – 300 |
| 24 | 0,01 – 0,03 | 150 – 300 |
| 26 | 0,03 – 0,1 | >800 |
| 28 | 0,1 – 1,0 | 150 – 300 |

*2. Thrombocytenaggregationshemmende Wirkung*
nach G. V. R. Born, Nature 4832, 927—929 (1962) und K. Breddin et al., Klin. Wschr. *53*, 81—89 (1975)

In plättchenreichem Plasma (PRP) von Beagle-Hunden wird durch in vitro-Gabe von Adenosindiphosphat (ADP) in einer Endkonzentration von 2,5 x $10^{-6}$ g/ml experimentell eine Thrombocytenaggregation induziert, die mit Hilfe eines Universalaggregometers der Firma B. Braun/Melsungen gemessen wird. Hierbei registriert ein Eppendorf-Photometer die Änderung der optischen Dichte des Plasmas.

Folgende Parameter werden erfaßt:
1) Der Winkel $\alpha$ zwischen der Tangente am Anfangsteil der kurve udn der waagerechten Tangente
2) Die Zeit Tr vom Beginn der Rotation (Aggregation) bis zur völligen Desaggregation
3) Die Maximalamplitude (Ma) der Aggregationskurve als Differenz zwischen der Ausgangstransmission und der maximalen Transmission.

TABELLE II

| Substanz Nr. | Endkonzentration in g/ml | $\alpha$ | Tr min | Ma | Änderung der Ma in % |
|---|---|---|---|---|---|
| 8 | $10^{-5}$ | 86,5 | 4,6 | 535 | –11 |
| (n = 5) | 3 × $10^{-5}$ | 86.6 | 4,3 | 523 | –13 |
| | $10^{-4}$ | 87,0 | 4,0 | 483 | –20 |
| 14 | $10^{-4}$ | 86,3 | 1,8 | 315 | –42 |
| (n = 5) | | | | | |
| 15 | $10^{-4}$ | 85,9 | 2,3 | 344 | –18 |
| (n = 5) | | | | | |
| 16 | $10^{-4}$ | 85,8 | 3,0 | 328 | –19 |
| (n = 5) | | | | | |
| Bencyclan | $10^{-4}$ | 85,2 | 2,6 | 324 | –34 |
| (n = 20) | | | | | |

*3. Akute Toxizität*

In Tabelle III sind für einige Verbindungen die an Mäusen und Ratten bei intraperitonealer und intravenöser Applikation über innerhalb von 7 Tagen auftretende Mortalität als $LD_{50}$ bzw. $LD_{50}$-Bereich bestimmte Toxizität angegeben.

TABELLE III

| Substanz Nr. | Applikationsart | $LD_{50}$ in mg/kg *) | |
|---|---|---|---|
| | | Maus | Ratte |
| 8 | i.v. | 83 (75,7–91) | 60 (53–67) **) |
| | i.p. | 100 – 300 **) | |
| 10 | i.p. | 100 – 300 | |
| 14 | i.v. | 40 – 63 | |
| | i.p. | 200 – 400 | |
| 15 | i.v. | 54 (51–57) | 25 – 40 |
| 16 | i.v. | 100 – 150 | 66 (63–69) |
| Pizotifen-HCl ***) | i.v. | 43 ± 1,8 | 17 ± 1,1 |

*) berechnet nach Litchfield u, Wilcoxon, J. Pharmacol, exp. Ther 96, 99 (1949).

**) für die Base.

***) Speigt, T. M. and G. S. Avery, Drugs, 3, 159 – 203 (1972).

Wie aus den vorstehenden Tabellen hervorgeht, sind die erfindungsgemäß erhaltenen Verbindungen größtenteils dem Vergleichspräparat Pizotifen-HCl überlegen. Darüber hinaus haben tierexperimentelle Untersuchungen weitere Vorzüge gegenüber Pizotifen-HCl in der Weise gezeigt, daß a) die Wirkung wesentlich schneller eintritt, b) im therapeutischen Dosisbereich keine Sedation hervorgerufen und c) keine unerwünschte Appetitstimulation bewirkt wird.

Eine besondere Anwendung der erfindungsgemäß erhaltenen Verbindungen entsprechend der Formel (I) sowie deren Salze liegt in der Kombination mit anderen geeigneten Wirkstoffen, wie Analgetica, Ergotamin-Präparaten, anderen Serotoninantagonisten, Spasmolytica, Vasodilatantien, $\beta$-Sympatholytica, Antemetica, Antihistaminica, Sedativa, Tranquillatien, Coffein, Nicotinsäure-Derivaten, Vitaminen und Östrogenen.

Beispiel 1

3 - [3 - (4 - Phenyl - piperazino) - propyl] - 6,6 - dimethyl - dihydrouracil - hydrochlorid (Tabelle IV, Nr. 8)

A) 11,0 g (0,05 mol) 1-Phenyl-4-(3-aminopropyl)-piperazin und 7,85 g (0,05 mol) 3-Isocyanatoisovaleriansäuremethylester werden nach Verfahrensvariante a) in je 50 ml Toluol gelöst, miteinander vermischt und kurz zum Sieden erhitzt. Nach dem Abkühlen wird das Lösungsmittel unter vermindertem Druck abdestilliert und der ölige Rückstand in Äther aufgenommen. Nach Zusatz von Petroläther und Reiben mit einem Glasstab erhält man die Verbindung II kristallin. Sie läßt sich aus einem Aceton-Petroläther-Gemisch umkristallisieren. Schmelzbereich 75 bis 77°C; Ausbeute 15,1 g (80,0% der Theorie).

Zur Überführung in das Hydrochlorid wird die Base in Azeton gelöst und mit der äquimolaren Menge 1n-Salzsäure versetzt. Man entfernt das Lösungsmittel unter vermindertem Druck bei maximal 30°C und kristallisiert den Rückstand aus Dioxan um, wodurch man das Zwischenprodukt 3 - [3 - (3 - [4 - Phenyl - piperazino] - propyl) - ureido] - isovaleriansäuremethylester - hydrochlorid in reiner

Form erhält. Ausbeute quantitativ; Schmelzbereich 168 bis 170°C; $C_{20}H_{33}ClN_4O_3$; Molekulargewicht 412,96.

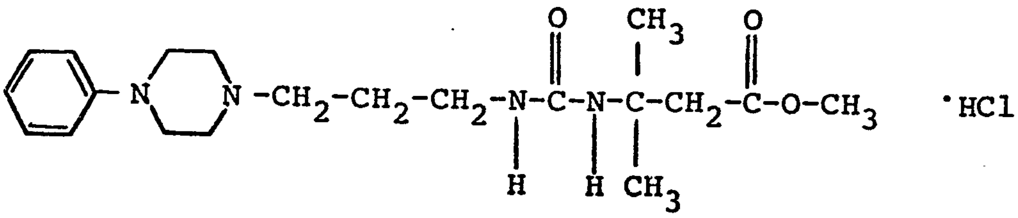

Analyse:

| | | | |
|---|---|---|---|
| berechnet: | C 58,17 | H 8,05 | N 13,57 |
| gefunden: | C 58,07 | H 7,97 | N 13,46 |

Zur Cyclisierung wird die Verbindung in etwa der 10-fachen Menge 12 %iger Salzsäure gelöst und zwei Stunden am Rückfluß gekocht, wobei innerhalb der letzten Stunde etwa die Hälfte des Lösungsmittels bei Normaldruck abdestilliert wird. Man dampft unter vermindertem Druck ein, löst den Rückstand in Wasser und fügt einen Überschuß wässeriger Natronlauge hinzu. Die ausgefallene Base wird in Methylenchlorid aufgenommen, über Natriumsulfat getrocknet und nach Eindampfen aus einem Methanol-Diisopropyläther-Gemisch umkristallisiert. Schmelzbereich 135 bis 136°C. Die Cyclisierung kann auch durchgeführt werden, indem man 5,65 g (0,015 mol) 3-[3-(3-{4-Phenyl-piperazino}-propyl)-ureido]-isovaleriansäuremethylester unter Stickstoffatmosphäre 1,5 Stunden auf 200°C erhitzt. Der Verlauf der Cyclisierung wird dabei dünnschichtchromatographisch verfolgt. Nach beendeter Reaktion wird die erstarrte Schmelze mit Diisopropyläther angerieben, über eine Chromatographiersäule gereinigt und aus Methanol-Diisopropyläther umkristallisiert. Ausbeute: 2,5 g (48% der Theorie); Schmelzbereich: 134 bis 136°C.

Zur Überführung in das Monohydrochlorid wird die Base in methanol gelöst und mit der berechneten Menge 1 n Salzsäure versetzt. Nach dem Eindampfen unter vermindertem Druck kristallisiert man den Rückstand aus Methanol oder Wasser um. Schmelzbereich 236 bis 242°C; Summenformel $C_{19}H_{29}ClN_4O_2$; Molekulargewicht: 380,92.

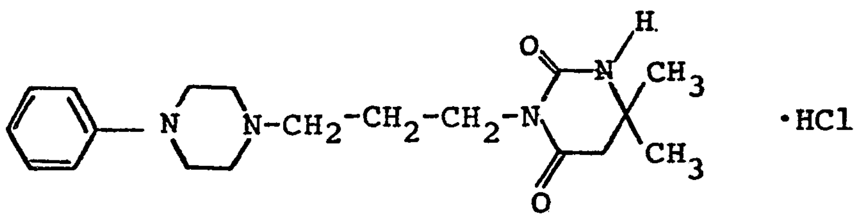

Analyse:

| | | | | |
|---|---|---|---|---|
| berechnet: | C 59,91 | H 7,67 | Cl 9,31 | N 14,71 |
| gefunden: | C 60,28 | H 7,75 | Cl 9,39 | N 14,73 |

B) Dieselbe Substanz kann auch wie folgt hergestellt werden:

Eine Lösung von 21,9 g (0,1 mol) 1-Phenyl-4-(3-aminopropyl)-piperazin in 100 ml Toluol wird nach Verfahrensweise a) unter Rühren mit einem Gemisch aus 15,7 g (0,1 mol) 3-Isocyanatoisovaleriansäuremethylester und 100 ml Toluol versetzt. Man erhitzt kurz zum Sieden, kühlt auf Raumtemperatur ab, dampft zur Trockne ein und löst den Rückstand in etwa 1 l Aceton. Nach Zusatz von 100 ml (0,1 mol) 1 n Natronlauge wird das Reaktionsgemisch 20 Stunden bei Raumtemperatur gerührt. Anschließend fügt man 100 ml (0,1 mol) 1 n Salzsäure hinzu, dampft zur Trockne ein, kristallisiert den Rückstand aus einem Azeton-Petroläther-Gemisch um und erhält so als Zwischenprodukt die freie 3-[3-(3-{4-Phenyl-piperazino}-propyl)-ureido]-isovaleriansäure. Ausbeute: 23,9 g (66% der Theorie); Schmelzbereich: 139 bis 140°C; Summenformel: $C_{19}H_{30}N_4O_3$; Molekulargewicht 362,47.

Analyse:

| | | | |
|---|---|---|---|
| berechnet: | C 62,96 | H 8,34 | N 15,46 |
| gefunden: | C 62,93 | H 8,30 | N 15,30 |

Das so erhaltene Zwischenprodukt wird 1 Stunde auf 180—200°C erhitzt. Nach dem Abkühlen wird die erstarrte Base säulenchromatographisch gereinigt und aus einem Methanol-Diisopropyläther-Gemisch umkristallisiert. Schmelzbereich: 135 bis 136°C.

Als weitere Variante kann die Cyclisierung der offenkettigen Carbonsäure zum entsprechenden 5,6-Dihydrouracil durch zweistündiges Erhitzen in wäßrig-alkoholischer Salzsäure, Acetylchlorid oder Essigsäureanhydrid erfolgen. Nach Entfernen des entsprechenden Kondensationsmittels (Dehydratisierungsmittels) unter vermindertem Druck wird der Rückstand in Wasser gelöst und mit überschüssiger Kalilauge versetzt. Die ausgefallene Base wird in Methylenchlorid aufgenommen, über Natriumsulfat getrocknet und nach dem Eindampfen des Extrationsmittels gegebenenfalls säulenchromatographisch gereinigt.

Beispiel 2

3 - [3 - (4 - Diphenylmethyl - piperazino) - propyl] - 6,6 - dimethyl - dihydrouracil (Tabelle IV, Nr. 26).

9,0 g (0,029 mol) 1-Diphenylmethyl-4-(3-aminopropyl)-piperazin werden in 30 ml Toluol gelöst und nach Verfahrensvariante a) mit einem Gemisch von 4,6 g (0,029 mol) 3-Isocyanatovaleriansäuremethylester und 20 ml Toluol unter Rühren versetzt. Man erhitzt 30 Minuten auf 70°C, dampft unter vermindertem Druck zur Trockne ein und reibt den öligen Rückstand nach Zusatz von Diäthyläther an. Der so erhaltene 3-(1-Diphenylmethylpiperazino-propylureido)-isovaleriansäuremethylester ist dünnschichtchromatographisch rein.

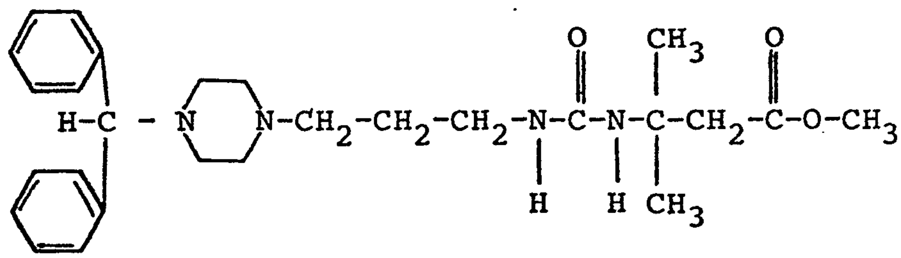

Ausbeute: 9,8 g (72% der Theorie)

9,8 g (0,021 Mol) des isolierten Esters werden in ca. 150 ml Methanol gelöst, mit 30 ml konzentrierter Salzsäure versetzt und 2 Stunden am Rückfluß erhitzt. Nach dem Entfernen des Lösungsmittels unter vermindertem Druck löst man den öligen Rückstand in Wasser und setzt die Base mit wässeriger Kalilauge frei. Man schüttelt mit Chloroform aus, trocknet die organische Phase über Natriumsulfat und dampft ein. Der kristalline Rückstand wird mehrmals mit Äther gewaschen.

Ausbeute: 7,1 g (77,8% der Theorie)
Schmelzbereich: 183—185°C
Summenformel: $C_{26}H_{34}N_4O_2$
Molekulargewicht: 434,58

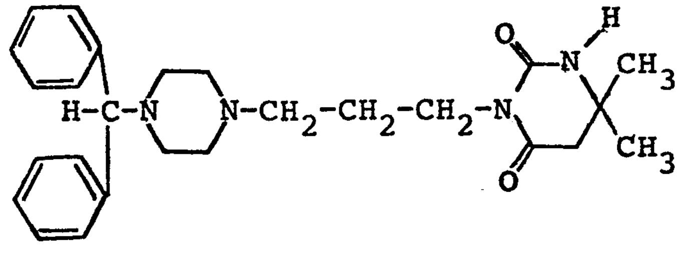

Analyse:

| | | | |
|---|---|---|---|
| berechnet: | C 71,86 | H 7,89 | N 12,89 |
| gefunden: | C 71,85 | H 8,05 | N 13,02 |

Die Verbindung kann anschließend nach den üblichen Methoden in die jeweils gewünschten Salze überführt werden.

Beispiel 3

3 - [2 - (4 - Phenyl - piperazino) - äthyl] - 5,6 - dimethyl - dihydrouracil - hydrochlorid (Tabelle IV, Nr. 20)

20,5 g (0,1 mol) 1-Phenyl-4-(2-aminoäthyl)-piperazin und 15,7 g (0,1 ml) 3-Isocyanato-2-methylbuttersäuremethylester werden nach Verfahrensvariante a) in je 125 ml Toluol gelöst, bei Raumtemperatur miteinander vermischt und 5 Minuten am Rückfluß erhitzt. Man engt unter vermindertem Druck ein, versetzt mit 200 ml 3 N Salzsäure und kocht 1 Stunde. Gegen Ende der Reaktionszeit wird etwa die Hälfte der Salzsäure unter Atmosphärendruck abdestilliert; der Rest wird anschließend unter vermindertem Druck entfernt. Der Rückstand kristallisiert nach Anreiben mit Methanol und wird mehrmals aus Methanol-Wasser umkristallisiert. Ausbeute: 21,5 g (58,7% der Theorie); Schmelzbereich 265 bis 273°C; Summenformel $C_{18}H_{27}ClN_4O_2$; Molekulargewicht 366,89.

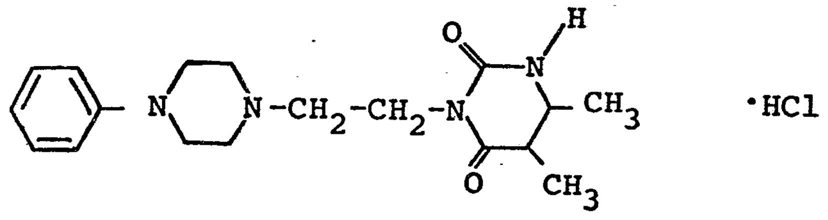

Analyse:

| | | | | |
|---|---|---|---|---|
| berechnet: | C 58,93 | H 7,42 | Cl 9,66 | N 15,27 |
| gefunden: | C 58,73 | H 7,53 | Cl 9,70 | N 15,16 |

Beispiel 4

3 - [3 - (4 - Phenyl - piperazino) - 6,6 - dimethyl - 2 - thiodihydrouracil - hydrochlorid (Tabelle IV, Nr. 14)

Eine Lösung von 21,9 g (0,1 mol) 1-Phenyl-4-(3-aminopropyl)-piperazin in 100 ml Toluol wird nach Verfahrensvariante a) unter Rühren mit einem Gemisch aus 17,3 g (0,1 mol) 3-Isothiocyanatoisovaleriansäuremethylester und 100 ml Toluol versetzt. Das Reaktionsgemisch wird 15 Minuten am Rückfluß erhitzt und anschließend unter vermindertem Druck vom Lösungsmittel befreit.

Der Eindampfrückstand wird auf einer Kieselgel 60-Säule (Firma E. Merck) (Durchmesser: 6 cm, Höhe: 70 cm) mit einem Chloroform-Methanol-Gemisch (Volumenverhältnis 9:1) gereinigt.
Ausbeute 19,1 g (53% der Theorie)
Schmelzbereich: 150—151°C.

Zur Überführung in das Hydrochlorid werden die zuvor erhaltenen 19,1 g Base in Methanol gelöst und mit 53 ml N Salzsäure versetzt. Nach dem Eindämpfen erhält man einen festen kristallinen Rückstand, der sich aus Methanol umkristillisieren läßt.
Schmelzbereich 200—217°C, Summenformel $C_{19}H_{29}ClN_4OS$, Molekulargewicht 396,98

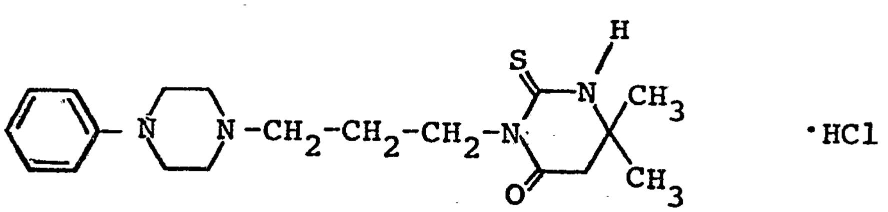

Analyse:

| | | | | | |
|---|---|---|---|---|---|
| berechnet: | C 57,49 | H 7,36 | Cl 8,93 | N 14,11 | S 8,08 |
| gefunden: | C 57,71 | H 7,46 | Cl 8,96 | N 14,10 | S 8,22 |

Beispiel 5

3 - [2 - (4 - Phenyl - piperazino) - äthyl] - 6,6 - dimethyl - 2 - thio - dihydrouracil - hydrochlorid (Tabelle IV, Nr. 16)

Einer Lösung von 25,8 g (0,145 mol) N,N'-Thiocarbonyldi-imidazol in 500 ml wasserfreiem Tetrahydrofuran werden nach Verfahrensvariante b) 29,8 g (0,145 mol) 1-Phenyl-4-(2-aminoäthyl)-piperazin in 125 ml wasserfreiem Tetrahydrofuran innerhalb von 90 Minuten unter Rühren tropfenweise zugesetzt. Man rührt 90 Minuten nach und fügt anschließend ebenfalls unter Rühren 24,3 g (0,145) mol) wasserfreies 3-Aminoisovaleriansäuremethylester-hydrochlorid hinzu. Die klare Lösung wird 120 Minuten am Rückfluß gekocht. Nach dem Abkühlen auf Raumtemperatur wird eine Lösung von 3,33 g (0,145 Grammatom) Natrium in 200 ml Methanol zugesetzt und 15 Minuten nachgerührt. Man entfernt das Lösungsmittel unter vermindertem Druck und wäscht den kristallinen Rückstand mehrmals mit Wasser. Nach dem Trocknen wird die Base mit Diäthyläther gewaschen und aus Äthanol umkristallisiert.

Ausbeute: 35,7 g (71% der Theorie) Schmelzbereich. 185—188°C.

Zur Herstellung des Monohydrochlorids wird die Base in Methylenchlorid gelöst und mit einem Überschuß äthanolischer Salzsäure versetzt. Nach Zusatz von Diäthyläther erhält man eine farblose kristalline Substanz, die zweimal aus Äthanol umkristallisiert wird und das Monohydrochlorid darstellt. Der pH-Wert einer 0,1%igen wässerigen Lösung liegt bei 4,3. Schmelzbereich 239 bis 240°C; Summenformel: $C_{18}H_{27}ClN_4OS$; Molekulargewicht 382,95.

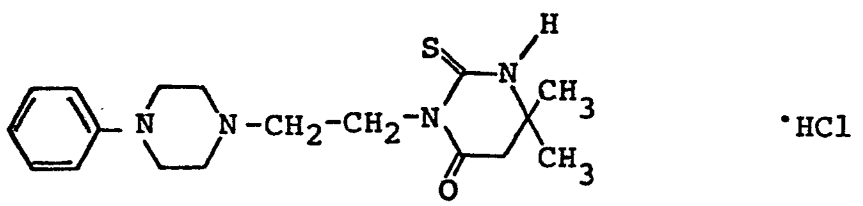

Analyse:

| | | | | | |
|---|---|---|---|---|---|
| berechnet: | C 56,46 | H 7,11 | Cl 9,26 | N 14,63 | S 8,37 |
| gefunden: | C 56,48 | H 7,11 | Cl 9,60 | N 14,62 | S 8,67 |

Zur Herstellung des Dihydrochloridhydrats wird die Base in wenig wasserhaltigem Methanol gelöst und mit einem Überschuß alkoholischer Salzsäure versetzt. Nach dem Ausfällen mit Diäthyläther erhält man die gewünschte Verbindung, die unter vermindertem Druck bei Raumtemperatur von anhaftender Salzsäure befreit und über Kalziumchlorid getrocknet wird. Der pH-Wert einer 10%igen wässerigen Lösung liegt bei 1,8. Schmelzbereich 238 bis 247°C unter Zersetzung; Summenformel $C_{18}H_{30}Cl_2N_4O_2S$; Molekulargewicht 437,42.

Analyse:

| | | | | | |
|---|---|---|---|---|---|
| berechnet: | C 49,43 | H 6,91 | Cl 16,21 | N 12,81 | S 7,33 |
| gefunden: | C 49,94 | H 6,95 | Cl 16,71 | N 12,90 | S 7,35 |

Beispiel 6

3 - [3 - (4 - Phenyl - piperazino) - propyl] - 1,6,6 - trimethyl - dihydrouracil - hydrochlorid (Tabelle IV, Nr. 23)

Einer Lösung von 16,2 g (0,1 mol) N,N'-Carbonyl-di-imidazol in 500 ml wasserfreiem Tetrahydrofuran werden nach Verfahrensvariante b) 21,9 g (0,1 mol) 1-Phenyl-4-(3-aminopropyl)-piperazin in 150 ml wasserfreiem Tetrahydrofuran innerhalb von 90 Minuten unter Rühren tropfenweise zugesetzt. Man rührt 30 Minuten nach und fügt ebenfalls unter Rühren 18,1 g (0,1 mol) 3-Methylaminoisovaleriansäuremethylesterhydrochlorid und 100 ml wasserfreies Dimethylformamid hinzu. Nach 180 Minuten Kochen am Rückfluß kühlt man auf Raumtemperatur ab, setzt eine Lösung von 2,29 g (0,1 Grammatom) Natrium in 50 ml Methanol zu und rührt weitere 15 Minuten nach. Nach Entfernen des Lösungsmittels unter vermindertem Druck wird der ölige Rückstand in Diäthyläther aufgenommen. Man filtriert von ungelösten Teilen ab, dampft die ätherische Lösung unter vermindertem Druck ein und versetzt den Rückstand mit wenig Methanol, worauf sich die gewünschte Base in kristalliner Form abscheidet. Die Verbindung läßt sich aus Methanol umkristallisieren. Ausbeute: 24,7 g (68,9% der Theorie).

Zur Überführung in das Hydrochlorid wird die Base in Azeton gelöst und mit der äquivalenten Menge 1 n Salzsäure versetzt. Nach dem Eindampfen unter vermindertem Druck wird der kristalline Rückstand aus Methanol mit Diäthyläther umgefällt. Der pH-Wert einer 0,1%igen Lösung liegt bei 3,0.

Schmelzbereich: 188—195°C

Summenformel: $C_{20}H_{31}ClN_4O_2$

Molekulargewicht: 394,94

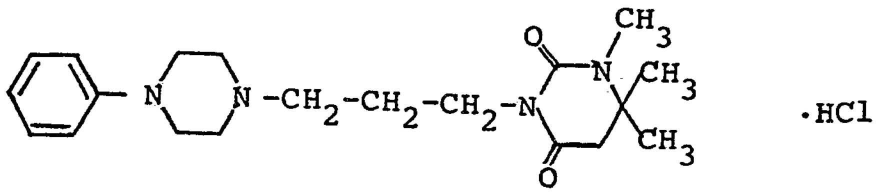

Analyse:

| | | | | |
|---|---|---|---|---|
| berechnet: | C 60,82 | H 7,91 | Cl 8,98 | N 14,19 |
| gefunden: | C 60,39 | H 7,87 | Cl 9,80 | N 13,69 |

TABELLE IV

| Nr. | Produkt | Schmelzbereich °C +) | gemäß Beispiel |
|---|---|---|---|
| 1 | 3-[3-(4-{3-Methylphenyl}-piperazino)-propyl]-6,6-dimethyl-dihydrouracil-hydrochlorid | 193–197° (122–123°) | 2 |
| 2 | 3-[3-(4-{2-Methoxyphenyl}-piperazino)-propyl]-6,6-dimethyl-dihydrouracil-hydrochlorid | 215–230° (122–123°) | 2 |
| 3 | 3-[3-(4-{2-Methylphenyl}-piperazino)-propyl]-6,6-dimethyl-dihydrouracil-hydrochlorid | 235–270° (116–118°) | 2 |
| 4 | 3-[3-(4-{3-Methoxyphenyl}-piperazino)-propyl]-6,6-dimethyl-dihydrouracil-hydrochlorid | 175–178° (92–94°) | 2 |
| 5 | 3-[3-(4-{4-Methoxyphenyl}-piperazino)-propyl]-6,6-dimethyl-dihydrouracil-hydrochlorid | 233–240° (163–167°) | 2 |
| 6 | 3-[3-(4-{3,4-Dimethylphenyl}-piperazino)-propyl]-6,6-dimethyl-dihydrouracil-hydrochlorid | 230–240° (162–163°) | 2 |
| 7 | 3-[3-(4-{2,6-Dimethylphenyl}-piperazino)-propyl]-6,6-dimethyl-dihydrouracil-hydrochlorid | 243–260° (121–125°) | 2 |
| 8 | 3-[3-(4-Phenyl-piperazino)-propyl]-6,6-dimethyl-dihydrouracil-hydrochlorid | 236–242° (135–136°) | 1 |
| 9 | 3-[3-(4-{1-Naphthyl}-piperazino)-propyl]-6,6-dimethyl-dihydrouracil-hydrochlorid | 265–285° | 2 |
| 10 | 3-[3-(4-{4-Fluorphenyl}-piperazino)-propyl]-6,6-dimethyl-dihydrouracil-hydrochlorid-hydrat | 198–200° (140–141°) | 2 |
| 11 | 3-[2-(4-Phenyl-piperazino)-äthyl]-6,6-dimethyl-dihydrouracil-hydrochlorid | 240–256° (140–144°) | 2 |
| 12 | 3-[2-(4-{2-Methoxyphenyl}-piperazino)-äthyl]-6,6-dimethyl-dihydrouracil-hydrochlorid | 230–242° | 3 |

+) Die Schmelzbereiche wurden mit dem Kofler-Heiztisch bestimmt und sind unkorrigiert.
Die Schmelzbereiche der entsprechenden Basen sind in Klammern angegeben.

TABELLE IV (Fortsetzung)

| Nr. | Produkt | Schmelzbereich °C | gemäß Beispiel |
|---|---|---|---|
| 13 | 3-[5-(4-{4-Methoxyphenyl}-piperazino)-pentyl}-6,6-dimethyl-dihydrouracil-hydrochlorid 1,5 $H_2O$ | 153–157° | 3 |
| 14 | 3-[3-(4-Phenyl-piperazino)-propyl]-6,6-dimethyl-2-thio-dihydrouracil-hydrochlorid | 200–217°<br>(150–151°) | 4 |
| 15 | 3-[3-(4-Phenyl-piperazino)-propyl]-5,6-dimethyl-dihydrouracil-hydrochlorid | 216–227° | 2 |
| 16 | 3-[2-(4-Phenyl-piperazino)-äthyl]-6,6-dimethyl-2-thio-dihydrouracil-hydrochlorid | 239–240°<br>(185–188°) | 5 |
| 17 | 3-[4-(4-Phenyl-piperazino)-butyl]-6,6-dimethyl-dihydrouracil-hydrochlorid-hydrat | 204–216°<br>(146–147°) | 2 |
| 18 | 3-[5-(4-Phenyl-piperazino)-pentyl]-5,6-dimethyl-dihydrouracil-hydrochlorid | 198–204° | 2 |
| 19 | 3-[5-(4-Phenyl-piperazino)-pentyl]-6,6-dimethyl-dihydrouracil-hydrochlorid | 187–193°<br>(109–110°) | 2 |
| 20 | 3-[2-(4-Phenyl-piperazino)-äthyl]-5,6-dimethyl-dihydrouracil-hydrochlorid | 265–273° | 3 |
| 21 | 3-[2-(4-Phenyl-piperazino)-äthyl]-5,6-dimethyl-2-thio-dihydrouracil-hydrochlorid | 244–250° | 5 |
| 22 | 3-[2-(4-Phenyl-piperazino)-äthyl]-1,6,6-trimethyl-2-thio-dihydrouracil-dihydrochlorid | 183–186° | 6 |
| 23 | 3-[3-(4-Phenyl-piperazino)-propyl]-1,6,6-trimethyl-dihydrouracil-hydrochlorid | 188–195° | 6 |
| 24 | 3-[3-(4-Phenyl-piperidino)-propyl]-6,6-dimethyl-dihydrouracil-hydrochlorid | 207–210°<br>(125–126°) | 3 |
| 25 | 3-[3-(4-Phenyl-piperidino)-propyl]-5,6-dimethyl-dihydrouracil-hydrochlorid | 223–238° | 3 |
| 26 | 3-[3-(4-Diphenylmethyl-piperazino)-propyl]-6,6-dimethyl-dihydrouracil | (183–185°) | 2 |
| 27 | 3-[3-(4-Diphenylmethyl-piperazino)-propyl]-5,6-dimethyl-dihydrouracil | (195–196°) | 2 |
| 28 | 3-[3-(4-Phenyl-piperazino)-2-hydroxypropyl]-6,6-dimethyl-dihydrouracil-hydrochlorid | 220–234°<br>(unter Zersetzung) | 1 |

**Patentansprüche**

1. Dihydrouracile der Formel I

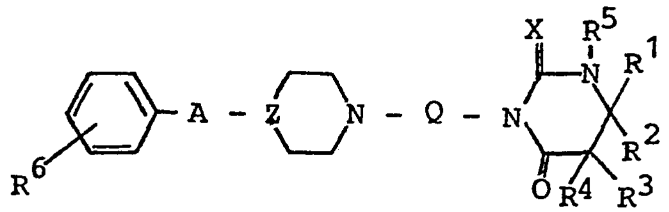

I

worin

$R^1$ Alkyl mit bis zu 2 C-Atomen,

$R^2$ bis $R^5$ Wasserstoff oder Alkyl mit zu 2 C-Atomen, wobei $R^2$ bis $R^5$ gleich oder verschieden sind,

$R^6$ Wasserstoff, einen anellierten Benzolring oder 1 bis 3 Substituenten der Gruppe Alkoxy mit 1 bis 3 C-Atomen, Halogen oder Alkyl mit 1 bis 4 C-Atomen,

A eine Einfachbindung oder die Gruppe

$$C_6H_5—\overset{|}{\underset{|}{C}}H,$$

Q Alkylen mit 2 bis 6 C-Atomen, wobei mindestens 2 C-Atome zwischen den beiden Stickstoffatomen stehen, oder die 2-Hydroxy-1,3-propylen-Gruppe,

X Sauerstoff oder Schwefel und

Z Stickstoff oder die Gruppe

$$—\overset{|}{\underset{|}{C}}H$$

und ihre physiologisch verträglichen Säureadditionssalze.

2. Verbindungen nach Anspruch 1, dadurch gekennzeichnet, daß

$R^1$ Alkyl mit bis zu 2 C-Atomen, .

$R^2$,$R^3$ Wasserstoff oder Alkyl mit 1 bis 2 C-Atomen,

$R^4$,$R^5$ Wasserstoff,

$R^6$ Wasserstoff oder Halogen,

A eine Einfachbindung,

Q eine unverzweigte Alkylenkette mit 2 bis 3 C-Atomen,

X Sauerstoff oder Schwefel und

Z Stickstoff bedeuten.

3. Verbindung nach Anspruch 2, dadurch gekennzeichnet, daß $R^1$ methyl, einer der Reste $R^2$ und $R^3$ Methyl und der andere Wasserstoff und $R^6$ Wasserstoff oder Fluor in 4-Stellung bedeuten.

4. 3 - [3 - (4 - {4 - Fluorphenyl} - 1 - piperazinyl) - propyl] - 6,6 - dimethyl - dihydrouracil - hydrochlorid - hydrat nach Anspruch 1.

5. 3 - [3 - (4 - Phenyl - 1 - piperazinyl) - propyl] - 6,6 - dimethyl - dihydrouracil - hydrochlorid nach Anspruch 1.

6. 3 - [3 - (4 - Phenyl - piperazinyl) - propyl] - 6,6 - dimethyl - 2 - thio - dihydrouracil - hydrochlorid nach Anspruch 1.

7. 3 - [3 - (4 - Phenyl - piperazinyl) - propyl] - 5,6 - dimethyl - dihydrouracil - hydrochlorid nach Anspruch 1.

8. 3 - [2 - (4 - Phenyl - 1 - piperazinyl) - äthyl] - 6,6 - dimethyl - 2 - thio - dihydrouracil - hydrochlorid nach Anspruch 1.

9. Verfahren zur Herstellung von Dihydrouracilen gemäß Anspruch 1, dadurch gekennzeichnet, daß man eine Verbindung der Formel II

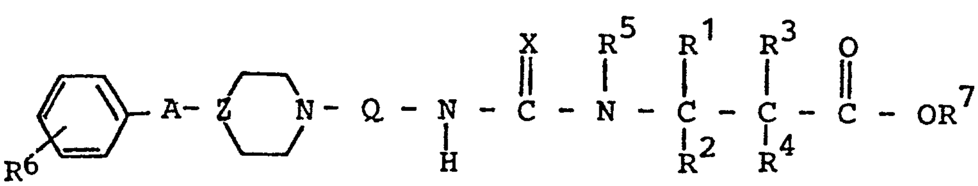

(II)

worin $R^1$ bis $R^6$, A, Q, Z und X die in Anspruch 1 genannten Bedeutungen haben und $OR^7$ Hydroxyl oder eine Alkoxygruppe mit 1 bis 4 C-Atomen bedeutet, unter Abspaltung von $R^7OH$ cyclisiert und gewünschtenfalls die Verbindungen der Formel I als solche isoliert oder mit Säuren in ihre physiologisch verträglichen Säureadditionssalze umwandelt.

10. Arzneimittel enthaltend mindestens eine Verbindung nach Anspruch 1 allein oder in Kombination mit üblichen Zusatzstoffen.

**Claims**

1. Dihydrouracils of formula I

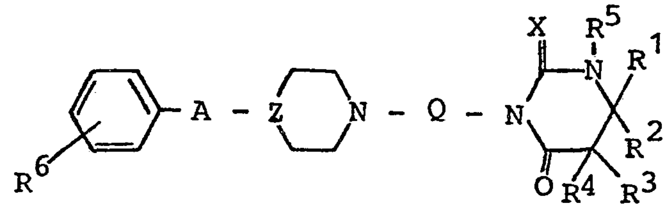

(I)

wherein

$R^1$ represents alkyl with up to 2 carbon atoms,

$R^2$ to $R^5$ each represent hydrogen or alkyl having up to 2 carbon atoms whereby $R^2$ to $R^5$ are identical or different,

$R^6$ represents hydrogen, an anellated benzene nucleus or 1 to 3 substituents from the group consisting of alkoxy groups having from 1 to 3 carbon atoms, halogen or alkyl having from 1 to 4 carbon atoms;

A represents a single bond or a

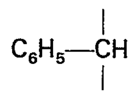

group;

Q represents alkylene with 2 to 6 carbon atoms at least two carbon atoms being between the two nitrogen atoms or the 2-hydroxy-1,3-propylene group

X is an oxygen or sulphur atom; and

Z is nitrogen or the group

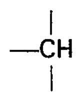

and the physiologically acceptable acid addition salts thereof.

2. Compounds as claimed in claim 1 in which

$R^1$ represents an alkyl group having up to 2 carbon atoms;

$R^2$ and $R^3$ each represent hydrogen or an alkyl with up to 2 carbon atoms;

$R^4$ and $R^5$ each represent hydrogen;

$R^6$ represents hydrogen or halogen;

A represents a single bond;

Q represents a straight-chained alkylene group having 2 to 3 carbon atoms;

X represents an oxygen or sulphur atom; and

Z represents a nitrogen atom.

3. Compounds as claimed in claim 2 wherein $R^1$ represents a methyl group; one of $R^2$ and $R^3$ represents a methyl group and the other hydrogen; and $R^6$ represents hydrogen or fluor in the 4-position.

4. 3 - [3 - (4 - {4 - Fluorphenyl} - 1 - piperazinyl) - propyl] - 6,6 - dimethyl - dihydrouracil - hydrochloride hydrate as claimed in claim 1.

5. 3 - [3 - (4 - Phenyl - 1 - piperazinyl) - propyl] - 6,6 - dimethyl - dihydrouracil - hydrochloride as claimed in claim 1.

6. 3 - [3 - (4 - Phenyl - 1 - piperazinyl) - propyl] - 6,6 - dimethyl - 2 - thio - dihydrouracil - hydrochloride as claimed in claim 1.

7. 3 - [3 - (4 - Phenyl - 1 - piperazinyl) - propyl] - 5,6 - dimethyl - dihydrouracil - hydrochloride as claimed in claim 1.

8. 3 - [2 - (4 - Phenyl - 1 - piperazinyl) - ethyl] - 6,6 - dimethyl - 2 - thio - dihydrouracil - hydrochloride as claimed in claim 1.

9. A process for the preparation of dihydrouracils as claimed in claim 1, characterised in cyclising a compound of formula II

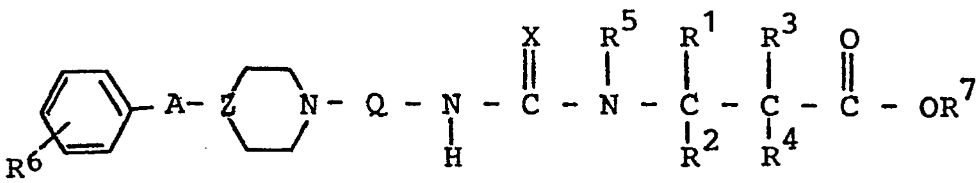 (II)

wherein $R^1$ to $R^6$, A, Q, Z and X represent the meanings as mentioned in claim 1 or $OR^7$ represents hydroxy or an alkoxy group with 1 to 4 carbon atoms, by elimination of $R^7OH$ and isolating the compounds of formula I as such, if desired, or converting them with acids into their physiologically acceptable acid addition salts.

10. Medicament containing at least one compound as claimed in claim 1 either alone or in combination with usual additives.

## Revendications

1. Dihydrouraciles de formule I

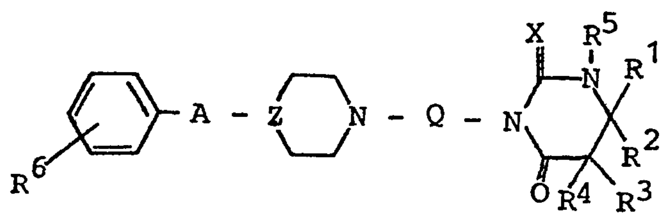 (I)

dans laquelle:

$R^1$ représente un radical alkyle ayant jusqu'à 2 atomes de carbone,

$R^2$ à $R^5$ représentent l'hydrogène ou un alkyle radical ayant jusqu'à 2 atomes de carbone, $R^2$ à $R^5$ étant identiques ou différents,

$R^6$ représente l'hydrogène, un noyau benzènique condensé ou de 1 à 3 substituants choisis parmi les radicaux alcoxy ayant de 1 à 3 atomes de carbone, les halogènes et les radicaux alkyle ayant jusqu'à 4 atomes de carbone;

A représente une simple liaison ou le groupe

$$C_6H_5—\overset{|}{\underset{|}{C}}H,$$

Q représente un radical alkylène ayant de 2 à 6 atomes de carbone, avec au moins 2 atomes de carbone intercalés entre les deux atomes d'azote, ou le groupe 2-hydroxy-1,3-propylène,

X représente l'oxygène ou le soufre, et

Z représente l'azote ou le groupe

$$—\overset{|}{\underset{|}{C}}H$$

et leurs sels d'addition avec des acides physiologiquement acceptables.

2. Composés selon la revendication 1, caractérisés en ce que:

$R^1$ représente un radical alkyle ayant jusqu'à 2 atomes de carbone,

$R^2$, $R^3$ représentent l'hydrogène ou un radical alkyle ayant jusqu'à 2 atomes de carbone,

$R^4$, $R^5$ représentent l'hydrogène,

$R^6$ représente l'hydrogène ou un halogène

A représente une simple liaison

Q représente une chaîne alkylène non-ramifiée ayant de 2 à 3 atomes de carbone,

X représente l'oxygène ou le soufre, et

Z représente l'azote.

3. Composé selon la revendication 2, caractérisé en ce que $R^1$ représente un groupe méthyle, un

des radicaux $R^2$ et $R^3$ représente un groupe méthyle et l'autre l'hydrogène et $R^6$ représente l'hydrogène ou le fluor en position 4.

4. Chlorhydrate hydraté de 3 - [3 - (4 - (4 - fluorphényl) - 1 - pipérazinyl) - propyl] - 6,6 - diméthyl - dihydrouracile, selon la revendication 1.

5. Chlorhydrate de 3 - [3 - (4 - phényl - 1 - pipérazinyl) - propyl] - 6,6 - diméthyl - dihydrouracile, selon la revendication 1.

6. Chlorhydrate de 3 - [3 - (4 - phényl - pipérazinyl) - propyl] - 6,6 - diméthyl - 2 - thio - dihydrouracile, selon la revendication 1.

7. Chlorhydrate de 3 - [3 - (4 - phényl - pipérazinyl) - propyl] - 5,6 - diméthyl - dihydrouracile, selon la revendication 1.

8. Chlorhydrate de 3 - [2 - (4 - phényl - 1 - pipérazinyl) - éthyl] - 6,6 - diméthyl - 2 - thio - dihydrouracile, selon la revendication 1.

9. Procédé de préparation des dihydrouraciles selon la revendication 1, caractérisé en ce qu'on cyclise un composé de formule II

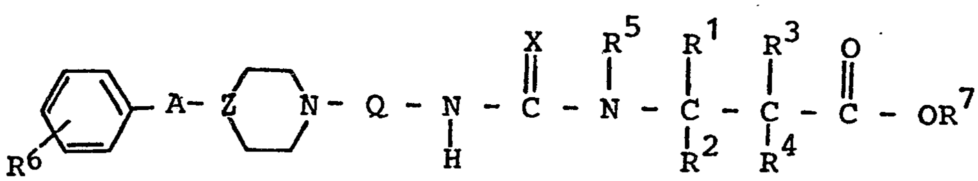

(II)

dans laquelle $R^1$ à $R^6$, A, Q, Z et X ont les significations indiquées dans la revendication 1, et $OR^7$ représente un hydroxyle ou un radical alcoxy ayant de 1 à 4 atomes de carbone, avec séparation de $R^7OH$, et éventuellement on isole les composés de formule I tels que ou on les convertit avec des acides en leurs sels d'addition avec des acides physiologiquement acceptables.

10. Médicament, caractérisé en ce qu'il contient au moins un composé selon la revendication 1, seul ou associé à des additifs usuels.